# EUROPÄISCHE PATENTSCHRIFT

(11) **EP 3 560 409 B1**
(45) Veröffentlichungstag und Bekanntmachung des Hinweises auf die Patenterteilung: **26.10.2022**
(21) Anmeldenummer: 19169706.9
(22) Anmeldetag: 17.04.2019
(51) Int. Cl.: A61B 1/00

(54) **ANORDNUNG ZUR STERILEN HANDHABUNG EINES NICHT-STERILEN ENDOSKOPS IN EINER STERILEN UMGEBUNG**
ARRANGEMENT FOR STERILE HANDLING OF A NON-STERILE ENDOSCOPE IN A STERILE ENVIRONMENT
DISPOSITIF POUR LA MANIPULATION STÉRILE D'UN ENDOSCOPE NON STÉRILE DANS UN ENVIRONNEMENT STÉRILE

(30) Priorität: 26.04.2018 DE 102018110082
(43) Veröffentlichungstag der Anmeldung: 30.10.2019
(73) Patentinhaber: avateramedical GmbH, 07745 Jena (DE)
(72) Erfinder: Beyer, Stefan, 10969 Berlin (DE); Weise, Fabian, 10437 Berlin (DE)
(74) Vertreter: Schaumburg und Partner Patentanwälte mbB

(56) Entgegenhaltungen:
- EP-A1- 0 520 743
- WO-A2-00/59366
- DE-A1-102016 007 669
- US-A- 5 257 617
- US-A1- 2005 159 646
- US-A1- 2014 200 406

## Beschreibung

Die Erfindung betrifft eine Anordnung zur sterilen Handhabung eines nicht sterilen Endoskops in einer sterilen Umgebung.

Bekannte Anordnungen zur sterilen Handhabung eines nicht sterilen Endoskops in einer sterilen Umgebung, beispielsweise einem Operationssaal, umfassen ein nicht steriles Endoskop und eine sterile Endoskophülle, wobei eine solche Endoskophülle auch als "drape" bezeichnet wird. Bei der Endoskophülle handelt es sich typischerweise um einen sterilen Einmalartikel oder um eine Endoskophülle, die wieder sterilisiert, d.h. wiederaufbereitet, werden kann. Eine solche Endoskophülle ist beispielsweise aus dem Dokument DE 10 2010 022 429 A1 bekannt. Die Endoskophülle hat zwei Hüllenteile, die lösbar und fluiddicht mechanisch miteinander verbunden sind. Dokument DE 10 2010 053 814 A1 offenbart ferner ein Endoskop für medizinische Zwecke, das in ein sterilisiertes Gehäuse eingesetzt werden kann.

Nachteilig bei bisher bekannten Endoskophüllen ist, dass nicht sichergestellt werden kann, ob das Endoskop korrekt in die Endoskophülle eingeführt und ob ein an dem distalen Ende der Endoskophülle angeordnetes optisches Element, beispielsweise ein Austrittsfenster, sowohl korrekt sitzt als auch nicht beschädigt worden ist. Wird ein Endoskop, das nicht korrekt in eine Endoskophülle eingeführt worden ist, zur Bildgebung bei einem Patienten genutzt, können Probleme bei der Bildgebung oder Übertragung auftreten, wodurch erhebliche Risiken für die Gesundheit des Patienten entstehen können.

DE 10 2016 007669 A1 offenbart eine Anordnung umfassend eine sterile Endoskopschutzhülle gemäß dem Oberbegriff des Anspruchs 1 und ein nicht steriles Endoskop.

US 2014/0200406 A1 offenbart ein System zur Vermeidung von Kondensation einer distal angeordneten Scheibe eines Endoskops. Das Endoskop umfasst Beleuchtungsoptik zum Leiten von Beleuchtungslicht und eine Beobachtungsoptik, die in einem inneren Gehäuse des Endoskops ausgebildet ist

Aus dem Dokument EP 0 904 725 A1 ist ein Endoskop mit einem auswechselbaren Schaft bekannt, der als steriler Einmalartikel ausgebildet ist. Nachteilig bei diesem Endoskop sind die vergleichsweise hohen Kosten, die durch das Auswechseln des Schafts bei jeder Anwendung entstehen.

Aus dem Dokument US 2014/0200406 A1 ist ein Endoskop bekannt, bei dem das Beschlagen eines distal, d.h. auf einer einem Patienten zugewandten Seite, angeordneten optischen Elements mithilfe von Infrarotlicht verhindert wird. Aus dem Dokument US 2014/0200406 A1 ist ferner ein Verfahren bekannt, bei dem mithilfe des Infrarotlichts das Vorhandensein des optischen Elements überprüft wird.

Dokument DE 34 33 889 A1 offenbart eine optische Anordnung zur Objekterkennung, mit einem Endoskop und einem Retroreflektor.

Ferner ist aus Dokument EP 0 820 250 B1 ein System zur endoskopischen Diagnose bekannt, das sowohl sichtbares als auch infrarotes Licht zur Bildgebung verwendet. Ausgehend von dem bekannten Stand der Technik ist es Aufgabe der Erfindung, eine Anordnung zur sterilen Handhabung eines nicht sterilen Endoskops in einer sterilen Umgebung anzugeben, bei der zuverlässig überprüft werden kann, ob eine Endoskophülle korrekt installiert ist.

Diese Aufgabe wird durch eine Anordnung mit den Merkmalen des Anspruchs 1 gelöst. Vorteilhafte Weiterbildungen sind in den abhängigen Ansprüchen angegeben.

Die Endoskophülle der erfindungsgemäßen Anordnung umfasst ein an einem distalen Ende der Endoskophülle angeordnetes optisches Element. Das optische Element weist ein lichtreflektierendes Element oder einen lichtreflektierenden Bereich auf, das bzw. der von einer Detektionslichtquelle emittiertes Detektionslicht als Reflexionslicht in Richtung eines proximalen Endes der Endoskophülle reflektiert. Im einfachsten Fall ist das optische Element als lichtdurchlässiges, das Licht nicht formendes Element, ausgebildet. Hierbei kann es sich um eine einfache Sichtscheibe handeln. Alternativ kann das optische Element als Linse oder Prisma ausgebildet sein. Das lichtreflektierende Element kann beispielsweise auf das optische Element aufgebracht, geklebt, oder mit dem optischen Element mechanisch verbunden sein. Alternativ sind das optische Element und das lichtreflektierende Element als ein Stück integral gebildet. Der lichtreflektierende Bereich kann beispielsweise durch einen Schliff des optischen Elements oder eine Oberflächenbehandlung, wie beispielsweise Bedampfen mit einem Metall oder einer dielektrischen Schicht, gebildet sein. Bei dem lichtreflektierenden Element bzw. dem lichtreflektierenden Bereich handelt es sich vorzugsweise um eine lichtreflektierende Beschichtung, die auf das optische Element aufgebracht ist. Hierdurch ist eine sichere und kostengünstige Überwachung der korrekten Anordnung des Endoskops in der Endoskophülle und der korrekten Anordnung des optischen Elements, beispielsweise eines Austrittsfensters, an der Endoskophülle möglich.

In diesem Dokument wird als distal eine dem Patienten zugewandte Richtung und als proximal eine dem Patienten abgewandte Richtung bezeichnet. Bei einem Bezug auf ein Element, einen Gegenstand oder eine Anordnung wird distal und proximal in Bezug auf die bestimmungsgemäße Lage des Elements, des Gegenstandes bzw. der Anordnung verwendet.

Da das Detektionslicht an dem optischen Element reflektiert wird, kann durch Detektion des Reflexionslichts das Vorhandensein des optischen Elements und damit das Vorhandensein der sterilen Endoskophülle sicher festgestellt werden. Hierdurch kann auch verhindert werden, dass ein nicht steriles Endoskop ohne die sterile Endoskophülle in den Patienten eingeführt wird. Durch Bestimmen optischer Charakteristika des Reflexionslichts, wie beispielsweise der Lichtintensität, können ferner Rückschlüsse auf die Lage des optischen Elements gezogen werden. Dies erlaubt es zum einen, zuverlässig die korrekte Installation der Endoskophülle festzustellen. Zum anderen gestattet es, Produktionsfehler oder Transportschäden festzustellen. Beispielsweise kann eine nicht korrekte Anordnung des Endoskops in der Hülle festgestellt werden, wenn sich beim Transport das optische Element gelöst oder die Endoskophülle verzogen hat. Erfolgt eine kontinuierliche Detektion des Reflexionslichts, können Beschädigungen im Betrieb, beispielsweise beim Reinigen während einer Operation festgestellt werden.

Durch das lichtreflektierende Element bzw. den lichtreflektierenden Bereich ist eine kostengünstige Lösung geschaffen, mit der zuverlässig überprüft werden kann, ob die Endoskophülle korrekt installiert bzw. ob das Endoskop korrekt in der Endoskophülle positioniert ist. Insbesondere kann der korrekte Sitz der Endoskophülle am Endoskop festgestellt werden. Das lichtreflektierende Element bzw. der lichtreflektierende Bereich führt zudem zu keinem Problem bei der Einhaltung regulatorischer Anforderungen, wie beispielsweise Hygienevorschriften sowie Vorschriften betreffend elektrische Sicherheit oder Temperaturverteilung.

Es ist vorteilhaft, wenn das lichtreflektierende Element oder der lichtreflektierende Bereich in wenigstens einem optischen Wellenlängenbereich außerhalb des Wellenlängenbereichs des Detektionslichts transparent ist. Insbesondere reflektiert das lichtreflektierende Element oder der lichtreflektierende Bereich Licht in einem Wellenlängenbereich innerhalb des Wellenlängenbereichs des Detektionslichts. Das optische Element ist für Licht in dem wenigstens einen optischen Wellenlängenbereich transparent oder weitestgehend transparent. Dies erhöht die optische Qualität des optischen Elements der Endoskophülle. Vorzugsweise ist das lichtreflektierende Element oder der lichtreflektierende Bereich in einem Wellenlängenbereich sichtbaren Lichts, d.h. insbesondere von 380 nm bis 780 nm, transparent. Dies erlaubt es, das optische Element, beispielsweise in Verbindung mit einem Endoskop, für die Bildgebung im optischen Bereich zu verwenden, ohne dass es dabei zu Beeinträchtigungen durch das lichtreflektierende Element bzw. den lichtreflektierenden Bereich bei der Bildgebung kommt.

Ferner ist es vorteilhaft, wenn das lichtreflektierende Element oder der lichtreflektierende Bereich Licht in einem ultravioletten (UV), d.h. zwischen 280 nm und 380 nm, infraroten (IR), d.h. zwischen 3 µm und 1 mm oder nahinfraroten (NIR), d.h. zwischen 780 nm und 3 µm, Wellenlängenbereich reflektiert. Hierdurch wird ausschließlich Licht mit einem Wellenlängenbereich außerhalb des sichtbaren Lichtes als Reflexionslicht verwendet. Dies vermindert den Einfluss von Umgebungslicht auf die Detektion des Reflexionslichts und erhöht somit die Zuverlässigkeit, mit der der korrekte Sitz des optischen Elements und damit der korrekte Sitz der Endoskophülle und festgestellt werden kann.

Vorzugsweise ist das lichtreflektierende Element oder der lichtreflektierende Bereich auf einer dem proximalen Ende der Endoskophülle zugewandten Seite des optischen Elements angeordnet. Insbesondere ist das lichtreflektierende Element oder der lichtreflektierende Bereich auf einer einem Innenraum der Endoskophülle zugewandten Seite des optischen Elements angeordnet. Hierdurch sind das lichtreflektierende Element bzw. der lichtreflektierende Bereich unempfindlich gegen äußere Einflüsse, insbesondere gegen Verschmutzungen und Flüssigkeiten, welche das Vermögen des lichtreflektierenden Elements, zur Reflektion des Detektionslichts, vermindern und dadurch eine Detektion des Reflexionslichts unzuverlässiger machen würden.

Die Erfindung betrifft eine Anordnung zur sterilen Handhabung eines nicht sterilen Endoskops in einer sterilen Umgebung. Die erfindungsgemäße Anordnung umfasst eine sterile Endoskophülle nach oben beschriebener Art oder nach einer vorteilhaften Weiterbildung und ein nicht steriles Endoskop. Das Endoskop umfasst einen Endoskopschaft, eine Beleuchtungsoptik zum Leiten von Beleuchtungslicht und eine Beobachtungsoptik zur Detektion und/oder Weiterleitung von Umgebungslicht, das in ein distales Ende des Endoskopschafts eintritt. Das Endoskop ist in der Endoskophülle aufgenommen und durch diese gegenüber der Umgebung steril abgeschirmt. Vorzugsweise bildet die Beobachtungsoptik wenigstens einen optischen Kanal.

Die Verwendung des nicht sterilen Endoskops in Verbindung mit der sterilen Endoskophülle ist eine kostengünstige Alternative zur Wiedersterilisierung von mehrfach benutzbaren Endoskopen oder zur Verwendung von Einmalendoskopen. Die Endoskophülle gestattet es ferner, sicher zu überprüfen, ob das Endoskop korrekt in der Endoskophülle installiert ist. Hierdurch lässt sich verhindern, dass das Endoskop mit einer nicht korrekt sitzenden oder ganz ohne die sterile Endoskophülle bei einer Operation verwendet wird, so dass erhebliche Risiken für die Gesundheit des Patienten entstehen. Beispielsweise kann ein Trokar bereitgestellt werden, der einen Zugang zum Situs, d.h. des eröffneten Operationsgebiets, des Patienten nur dann freigibt, wenn der korrekte Sitz der Endoskophülle festgestellt wurde. Damit ermöglicht die erfindungsgemäße Anordnung eine kostengünstige und sichere Handhabung eines nicht sterilen Endoskops in einer sterilen Umgebung.

Erfindungsgemäß hat Endoskop eine von der Beleuchtungsoptik und der Beobachtungsoptik verschiedene Detektionsoptik zum Leiten des von der Detektionslichtquelle emittierten Detektionslichts. Beispielsweise kann das Detektionslicht durch einen Lichtwellenleiter, insbesondere einer Glasfaser, mit einer Dicke, insbesondere mit einem Kerndurchmesser, zwischen 3 µm und 1 mm von der Detektionslichtquelle zu dem distalen Ende des Endoskopschafts geführt werden. Hierdurch können das lichtreflektierende Element bzw. der lichtreflektierende Bereich sicher mit dem Detektionslicht beleuchtet werden, sodass ausreichend Reflexionslicht reflektiert und damit eine zuverlässige Detektion des Reflexionslichts sichergestellt wird. Vorzugsweise ist das lichtreflektierende Element oder der lichtreflektierende Bereich derart angeordnet, dass sich bei bestimmungsgemäßem Gebrauch der Anordnung ein distales Ende der Detektionsoptik und das lichtreflektierende Element bzw. der lichtreflektierende Bereich einander gegenüberliegen.

Weiterhin ist es vorteilhaft, wenn die Beobachtungsoptik das Reflexionslicht leitet. Der durch die Beobachtungsoptik gebildete optische Kanal kann dazu genutzt werden, das Reflexionslicht von dem distalen Ende des Endoskops zu einem proximalen Ende des Endoskops zu leiten. Durch die Verwendung der Beobachtungsoptik entfällt die Notwendigkeit einen eigenen optischen Kanal für das Reflexionslicht vorzusehen. Hierdurch wird der Aufbau des Endoskops kompakter und das Endoskop lässt sich einfacher und kostengünstiger herstellen.

Ferner ist es vorteilhaft, wenn das Endoskop einen Strahlteiler aufweist, der das Detektionslicht in die Beleuchtungsoptik oder die Detektionsoptik einkoppelt. Besonders vorteilhaft ist es, wenn der Strahlteiler das Reflexionslicht aus der Detektionsoptik oder der Beobachtungsoptik auskoppelt, so dass es auf ein Sensorelement, insbesondere einen Photodetektor oder einen CCD-Sensor, trifft. Durch den Strahlteiler lassen sich das Sensorelement und die Detektionslichtquelle kompakt anordnen, wodurch ein effizienter Aufbau des Endoskops erreicht wird. Besonders vorteilhaft ist, wenn das Sensorelement, die Detektionslichtquelle und der Strahlteiler an dem distalen Ende des Endoskops angeordnet sind, besonders vorteilhaft ist diese Anordnung bei sogenannten Chip-On-Tip Endoskopen, d.h. Endoskopen, bei denen ein zur Bildgebung verwendete Bildsensor an dem distalen Ende des Endoskops angeordnet ist. Ferner erlaubt es der Strahlteiler zum Transport des Detektionslichts und des Reflexionslichts den gleichen optischen Kanal zu nutzen, so dass keine zusätzlichen optischen Kanäle vorgesehen werden müssen.

Weiterhin ist es vorteilhaft, wenn das Endoskop ein Sensorelement, insbesondere einen Photodetektor oder einen CCD-Chip, umfasst, welches das Reflexionslicht erfasst. Die Verwendung eines separaten Sensorelements zur Detektion des Reflexionslichts vermindert den Einfluss von Umgebungslicht auf die Detektion des Reflexionslichts. Dies erhöht die Zuverlässigkeit, mit der das Reflexionslicht detektiert wird.

Ferner ist es vorteilhaft, wenn die Beobachtungsoptik einen Bildsensor umfasst, der sowohl das in das distale Ende des Endoskopschafts eintretende Umgebungslicht als auch das Reflexionslicht erfasst. Der Bildsensor kann beispielsweise an dem distalen Ende des Endoskops angeordnet sein. Das Endoskop ist dann ein sogenanntes Chip-On-Tip Endoskop. Alternativ kann der Bildsensor in einem proximalen Teil des Endoskops, insbesondere in einem Endoskopkörper, angeordnet sein. Vorzugsweise umfasst das Endoskop eine Steuereinheit zur Weiterverarbeitung von durch den Bildsensor erfassten Daten.

Es ist besonders vorteilhaft, wenn die Anordnung eine Steuereinheit umfasst, welche nur in einem ersten Betriebsmodus durch den Bildsensor aufgenommene Bilder zur Bildanzeige weiterverarbeitet, und welche die Detektionslichtquelle derart steuert, dass die Detektionslichtquelle nur in einem zweiten Betriebsmodus das Detektionslicht emittiert, wobei der Bildsensor im zweiten Betriebsmodus das von dem lichtreflektierenden Element reflektierte Reflexionslicht detektiert. Hierdurch wird verhindert, dass es bei der Detektion der Endoskophülle störende Effekte im angezeigten Bild, beispielsweise Reflexe, oder bei der Bildverarbeitung erzeugt werden. Beispielsweise nimmt der Bildsensor 30 Bilder (frames) pro Sekunde auf, von denen nur 29 zur Bildanzeige weiterverarbeitet werden. Das nicht zur Bildanzeige weiterverarbeitete Bild wird zur Detektion des Reflexionslichts verwendet. Alternativ nimmt der Bildsensor 31 Bilder pro Sekunde auf, von denen eins zur Detektion des Reflexionslichts verwendet wird. Dies hat den Vorteil, dass 30 Bilder pro Sekunde zur Bildanzeige weiterverarbeitet und angezeigt werden, was für das menschliche Auge sehr natürlich und angenehm wirkt.

Es ist vorteilhaft, wenn die Detektionslichtquelle das Beleuchtungslicht emittiert und wenn das Detektionslicht aus dem Beleuchtungslicht ausgekoppelt wird. Durch die Verwendung einer einzigen Lichtquelle zur Erzeugung des Beleuchtungslichts und des Detektionslichts kann ein besonders kompakter Aufbau des Endoskops erreicht werden.

Es ist besonders vorteilhaft, wenn die Detektionslichtquelle Detektionslicht in einem ultravioletten (UV), infraroten (IR) oder nahinfraroten (NIR) Wellenlängenbereich emittiert. Hierdurch wird ausschließlich Licht mit einem Wellenlängenbereich außerhalb des sichtbaren Lichtes als Reflexionslicht verwendet. Dies vermindert den Einfluss von Umgebungslicht auf die Detektion des Reflexionslichts. Hierdurch lässt sich die korrekte Installation der Endoskophülle an dem Endoskop zuverlässig feststellen.

Ferner ist es vorteilhaft, wenn die Detektionslichtquelle kohärentes Detektionslicht emittiert. Insbesondere kann es sich bei dem kohärenten Detektionslicht um Laserlicht handeln. Durch die Verwendung von Laserlicht kann insbesondere durch Triangulationsrechnung der Abstand zwischen dem distalen Ende des Endoskops und dem optischen Element ermittelt werden. Dies erlaubt eine besonders genaue Bestimmung der Lage des optischen Elements in Bezug zu dem Endoskop, wodurch besonders zuverlässig der korrekte Sitz der Endoskophülle auf dem Endoskop ermittelt werden kann.

Bei dem Vorsehen von unterschiedlichen optischen Elementen in verschiedenen Endoskophüllen kann über die Wahl der Endoskophülle die Abbildung des mit Hilfe des Endoskops detektierten Lichts verändert werden, so dass die optischen Bildaufnahmeeigenschaften des Endoskops durch die Wahl der Hülle verändert werden können.

Bei dem Endoskop kann es sich um ein Monoendoskop, d.h. mit nur einem optischen Kanal, oder ein stereoskopisches Endoskop handeln. Die Beobachtungsoptik des stereoskopischen Endoskops weist einen zweiten optischen Kanal auf. Alternativ umfasst das stereoskopische Endoskop eine zweite Beobachtungsoptik, die den zweiten optischen Kanal bildet.

Vorzugsweise ist die Detektionslichtquelle in dem Endoskop angeordnet. Die Detektionslichtquelle ist beispielsweise durch eine LED gebildet. Insbesondere kann die Detektionslichtquelle an dem distalen Ende des Endoskops angeordnet sein. Besonders vorteilhaft ist dies, wenn es sich bei dem Endoskop um ein Chip-On-Tip Endoskop handelt. Alternativ kann die Detektionslichtquelle in einem proximalen Teil des Endoskops, insbesondere in einem Endoskopkörper, angeordnet sein.

Besonders vorteilhaft ist es, wenn die Detektionslichtquelle und das Sensorelement in Lock-In-Technik arbeiten. Hierzu wird das von der Detektionslichtquelle emittierte Detektionslicht mit einem bekannten Referenzsignal moduliert. Mithilfe eines sogenannten Lock-In-Verstärkers, der dem Sensorelement nachgeschaltet ist, lässt sich auf Grundlage des bekannten Referenzsignals das Reflexionslicht zuverlässig vom Hintergrundrauschen trennen. Somit wird ein geringes Signal-Rauschen-Verhältnis der Anordnung erreicht.

Der Abstand zwischen dem optischen Element und dem distalen Ende des Endoskops hat vorzugsweise einen Wert im Bereich zwischen 10 µm und 200 µm. Dies verhindert eine Beeinflussung der Detektion des Reflexionslichts durch das Umgebungslicht, wodurch eine zuverlässige Detektion des Reflexionslichts erfolgt.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Figur 1: eine Anordnung zur sterilen Handhabung eines nicht sterilen Endoskops in einer sterilen Umgebung mit einer sterilen Endoskophülle gemäß einem ersten Ausführungsbeispiel;
- Figur 2a: eine schematische Schnittdarstellung einer Anordnung mit einem stereoskopischen Endoskop, das eine von einer Beobachtungsoptik verschiedene Detektionsoptik hat, und mit einer sterilen Endoskophülle, die ein an einem distalen Ende angeordnetes optisches Element hat, gemäß einem zweiten Ausführungsbeispiel;
- Figur 2b: eine schematische Darstellung einer Vorderansicht des stereoskopischen Endoskops gemäß dem zweiten Ausführungsbeispiel nach Figur 2a;
- Figur 2c: eine schematische Darstellung eines optischen Elements der sterilen Endoskophülle des stereoskopischen Endoskops gemäß dem zweiten Ausführungsbeispiel nach Figur 2a;
- Figur 3a: eine schematische Schnittdarstellung einer Anordnung mit einem stereoskopischen Endoskop und einer sterilen Endoskophülle gemäß einem dritten Ausführungsbeispiel;
- Figur 3b: eine schematische Darstellung einer Vorderansicht des stereoskopischen Endoskops gemäß dem dritten Ausführungsbeispiel nach Figur 3a;
- Figur 3c: eine schematische Darstellung eines optischen Elements der sterilen Endoskophülle des stereoskopischen Endoskops gemäß dem dritten Ausführungsbeispiel nach Figur 3a;
- Figur 4a: eine schematische Schnittdarstellung einer Anordnung mit einem Monoendoskop und einer sterilen Endoskophülle gemäß einem vierten Ausführungsbeispiel;
- Figur 4b: eine schematische Darstellung einer Vorderansicht des Monoendoskops gemäß dem vierten Ausführungsbeispiel nach Figur 4a;
- Figur 4c: eine schematische Darstellung eines optischen Elements der sterilen Endoskophülle des Monoendoskops gemäß dem vierten Ausführungsbeispiel nach Figur 4a;
- Figur 5a: eine schematische Schnittdarstellung einer Anordnung mit einem stereoskopischen Endoskop und einer sterilen Endoskophülle gemäß einem fünften Ausführungsbeispiel;
- Figur 5b: eine schematische Darstellung einer Vorderansicht des stereoskopischen Endoskops gemäß dem fünften Ausführungsbeispiel nach Figur 5a;
- Figur 5c: eine schematische Darstellung eines optischen Elements der sterilen Endoskophülle des stereoskopischen Endoskops gemäß dem fünften Ausführungsbeispiel nach Figur 5a;
- Figur 6a: eine schematische Schnittdarstellung einer Anordnung mit einem Monoendoskop und einer sterilen Endoskophülle gemäß einem sechsten Ausführungsbeispiel;
- Figur 6b: eine schematische Darstellung einer Vorderansicht des Monoendoskops gemäß dem sechsten Ausführungsbeispiel nach Figur 6a;
- Figur 6c: eine schematische Darstellung eines optischen Elements der sterilen Endoskophülle des Monoendoskops gemäß dem sechsten Ausführungsbeispiel nach Figur 6a;
- Figur 7: ein Diagramm zur Erläuterung der Funktionsweise der Anordnung zur sterilen Handhabung eines nicht sterilen Endoskops gemäß dem vierten Ausführungsbeispiel nach den Figuren 4a bis 4c;
- Figur 8: ein Diagramm eines Wellenlängenbereichs von Detektionslicht der Anordnung zur sterilen Handhabung eines nicht sterilen Endoskops gemäß dem ersten Ausführungsbeispiel nach Figur 1; und
- Figur 9: eine schematische Schnittdarstellung des distalen Endes des Endoskops gemäß dem dritten Ausführungsbeispiel nach den Figuren 3a bis 3c.

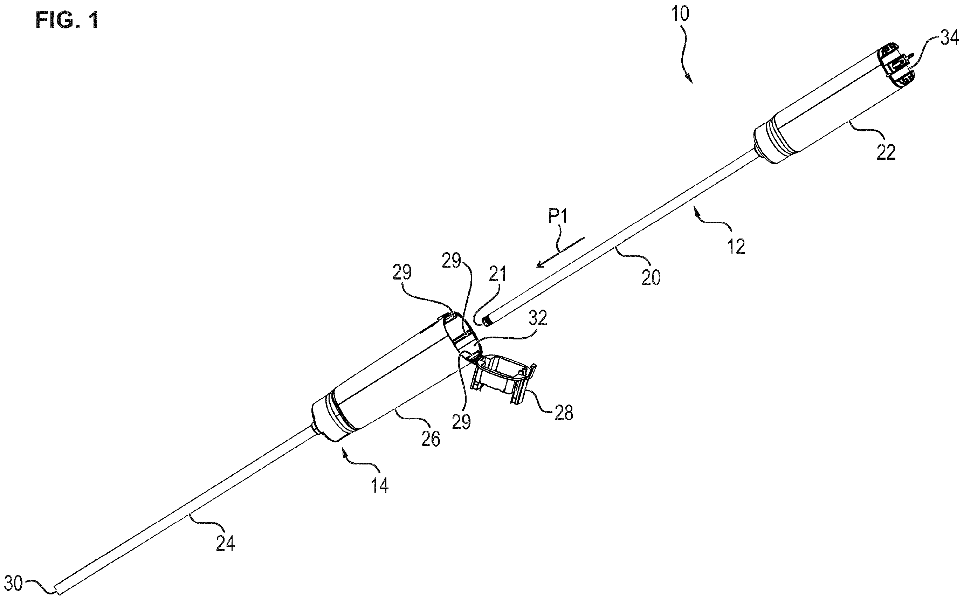

Figur 1 zeigt eine Anordnung 10 zur sterilen Handhabung eines nicht sterilen Endoskops 12 in einer sterilen Umgebung mit einer sterilen Endoskophülle 14 gemäß einem ersten Ausführungsbeispiel.

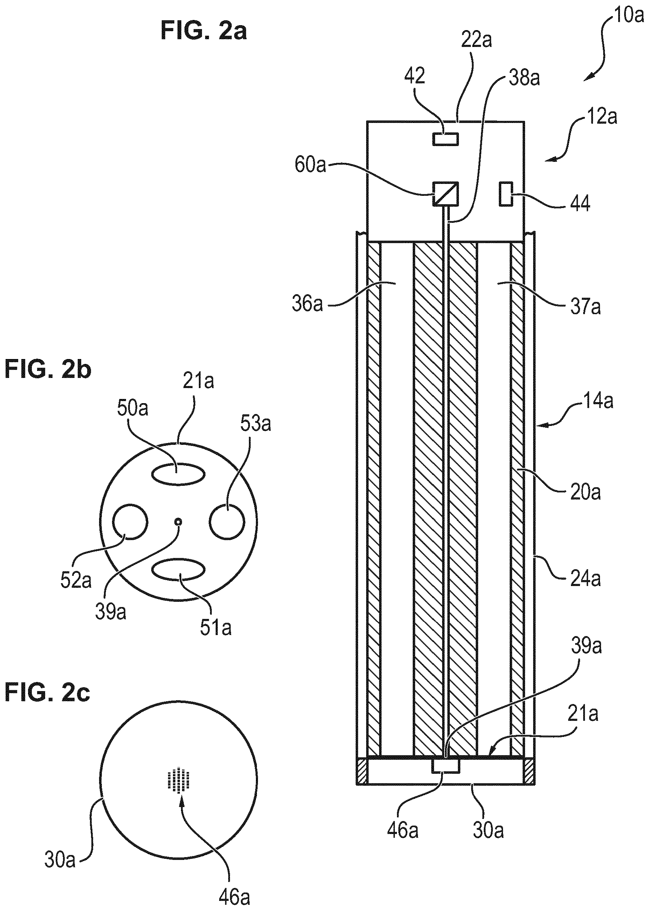

Das Endoskop 12 hat einen in distale Richtung weisenden Endoskopschaft 20 und einen an einem proximalen Ende des Endoskopschafts 20 angeordneten Endoskopkörper 22. Das Endoskop 12, insbesondere der innere Aufbau des Endoskops 12, wird nachfolgend anhand der Figuren 2a, 2b, 3a, 3b, 4a, 4b, 5a, 5b, 6a, 6b, 7a und 7b näher beschrieben.

Die Endoskophülle 14 umfasst einen vorderen Teil 24, der zur Aufnahme des zumindest teilweise in einen Körper eines Patienten einführbaren Endoskopschafts 20 dient. Der vordere Teil 24 der Endoskophülle 14 ist an dem distalen Ende mit Hilfe eines optischen Elements 30 verschlossen, das ein in Figur 1 nicht dargestelltes lichtreflektierendes Element 46 aufweist. Konkrete Ausführungsbeispiele des optischen Elements 30 werden nachfolgend in Verbindung mit den Figuren 2c, 3c, 4c, 5c, 6c und 7c näher beschrieben.

Die Endoskophülle 14 umfasst ferner ein Mittelteil 26 zur Aufnahme des Endoskopkörpers 22 und ein mit dem Mittelteil 26 verbundenes Verschlusselement 28 mit einer Sterilschleuse. Durch das Verschlusselement 28 ist eine Zuführ- und Entnahmeöffnung 32 der Endoskophülle 14 zum Einführen und Herausnehmen eines Endoskops in bzw. aus der Endoskophülle 14 gebildet. Mit Hilfe der Sterilschleuse ist ein Kontaktbereich 34 des Endoskops 12 mit elektrischen Kontakten und optischen Verbindungselementen steril abschirmbar.

Zur Aufnahme des Endoskops 12 in der Endoskophülle 14 wird das Endoskop 12 in Richtung des Pfeils P1 durch die geöffnete Zuführ- und Entnahmeöffnung 32 in die Endoskophülle 14 eingeführt. Hierzu wird der Endoskopschaft 20 zuerst in die Zuführ- und Entnahmeöffnung 32 eingeführt und nachfolgend bis in den vorderen Teil 24 der Endoskophülle 14 geschoben, so dass eine Spitze 21 des Endoskopschafts 20 dem an dem distalen Ende des vorderen Teils 24 angeordneten optischen Element 30 der Endoskophülle 14 gegenüberliegend angeordnet ist. Beim Einführen des Endoskopkörpers 22 durch die Zuführ- und Entnahmeöffnung 32 in den Mittelteil 26 der Endoskophülle 14 wird der Endoskopkörper 22 durch innen im Mittelteil 26 der Endoskophülle 14 vorhandene Führungsstege 29 geführt und in einer vorbestimmten Lage im Mittelteil 26 der Endoskophülle 14 gehalten.

Figur 2a zeigt eine schematische Schnittdarstellung einer Anordnung 10a gemäß einem zweiten Ausführungsbeispiel. Elemente mit gleichem Aufbau oder gleicher Funktion haben dieselben Bezugszeichen. Die Anordnung 10a umfasst ein stereoskopisches Endoskop 12a, das eine von einer Beobachtungsoptik 36a, 37a verschiedene Detektionsoptik 38a hat, und eine sterile Endoskophülle 14a, die ein an einem distalen Ende angeordnetes optisches Element hat, in einer schematischen Schnittdarstellung. Das nicht sterile Endoskop 12a ist in der sterilen Endoskophülle 14a aufgenommen und dadurch gegenüber der Umgebung steril abgeschirmt.

Von der Endoskophülle 14a ist in Figur 2a nur ein vorderer Teil 24a gezeigt. Der weitere Aufbau und die Funktion der Endoskophülle 14a entspricht der Endoskophülle 14 nach Figur 1. Das distale Ende des vorderen Teils 24a der Endoskophülle 14a ist durch ein optisches Element 30a verschlossen, bei dem es sich um eine Glasscheibe handelt. Auf der proximalen Seite des optischen Elements 30a ist ein lichtreflektierendes Element 46a angeordnet. Das lichtreflektierende Element 46a ist derart angeordnet, dass es einem distalen Ende 39a der Detektionsoptik 38a gegenüberliegt.

Das Endoskop 12a hat einen sich in distaler Richtung erstreckenden Endoskopschaft 20a, der die Detektionsoptik 38a und die Beobachtungsoptik 36a, 37a umfasst. Das Endoskop 12a hat ferner einen an dem proximalen Ende des Endoskopschafts 20a angeordneten Endoskopkörper 22a, in dem insbesondere ein Strahlteiler 60a, eine Detektionslichtquelle 42 und ein Sensorelement 44 angeordnet sind.

Von der Detektionslichtquelle 42 ausgehendendes Detektionslicht wird durch den Strahlteiler 60a in die Detektionsoptik 38a eingekoppelt. Die Detektionsoptik 38a leitet das Detektionslicht zu einer an dem distalen Ende des Endoskopschafts 20a angeordneten Spitze 21a, wo es aus dem distalen Ende 39a der Detektionsoptik 38a austritt. Das austretende Detektionslicht wird durch das dem distalen Ende 39a der Detektionsoptik 38a gegenüberliegende lichtreflektierende Element 46a als Reflexionslicht in Richtung der Detektionsoptik 38a zurück reflektiert. Das Reflexionslicht wird durch die Detektionsoptik 38a von dem distalen Ende 39a der Detektionsoptik 38a bis zum Strahlteiler 60a leitet. Nachdem das Reflexionslicht durch den Strahlteiler 60a hindurchgetreten ist, fällt es auf das Sensorelement 44, welches das Reflexionslicht erfasst.

Die Beobachtungsoptik 36a, 37a umfasst zwei optischen Kanäle. Durch die zwei optischen Kanäle wird in der Spitze 21a des Endoskopschafts 20a einfallendes Umgebungslicht von der Spitze 21a zu dem proximalen Ende des Endoskops 12a geleitet. Hierdurch ist eine stereoskopische Beobachtung eines Bereichs distal der Spitze 21a möglich.

Figur 2b zeigt eine schematische Darstellung einer Vorderansicht des stereoskopischen Endoskops 12a gemäß dem zweiten Ausführungsbeispiel nach Figur 2a von einer distalen Seite her gesehen. Das distale Ende 39a der Detektionsoptik 38a ist mittig angeordnet. In der Darstellung der Figur 2b sind links und rechts, d.h. beiderseits, des distalen Endes 39a der Detektionsoptik 38a je ein Fenster 52a, 53a der Beobachtungsoptik 36a, 37a angeordnet. In der Darstellung der Figur 2b sind oberhalb und unterhalb des distalen Endes 39a der Detektionsoptik 38a je ein distales Ende 50a, 51a einer Beleuchtungsoptik zweikanalige zum Beleuchten des Bereichs distal der Spitze 21a angeordnet.

Figur 2c zeigt eine Draufsicht des optischen Elements 30a der sterilen Endoskophülle 14a des stereoskopischen Endoskops 12a gemäß dem zweiten Ausführungsbeispiel nach Figur 2a von einer proximalen Seite her gesehen. Das lichtreflektierende Element 46a ist mittig auf dem optischen Element 30a angeordnet und als eine lichtreflektierende Beschichtung ausgebildet, die auf das optische Element 30a aufgebracht worden ist.

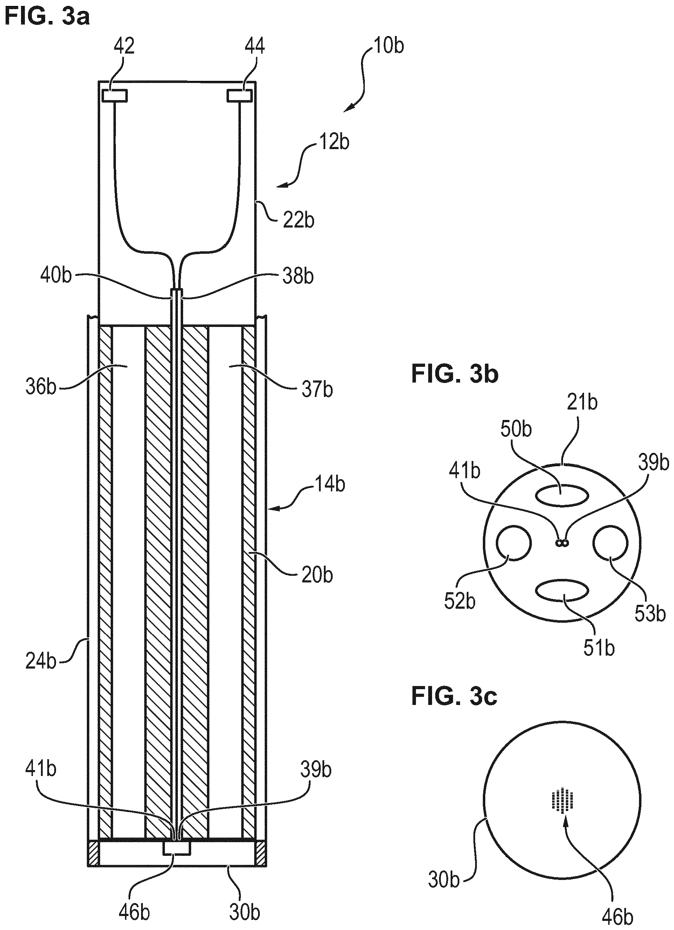

Figur 3a zeigt eine schematische Schnittdarstellung einer Anordnung 10b gemäß einem dritten Ausführungsbeispiel. Die Anordnung 10b umfasst ein stereoskopisches Endoskop 12b mit einem Endoskopkörper 22b, das die von einer Beobachtungsoptik 36b, 37b verschiedene Detektionsoptik und einen Lichtwellenleiter 40b zum Leiten von Detektionslicht hat. Ferner umfasst die Anordnung 10b eine sterile Endoskophülle 14b, die ein an dem distalen Ende angeordnetes optisches Element 30b hat. Die Anordnung 10b nach Figur 3 unterscheidet sich von der Anordnung 10a nach den Figuren 2a bis 2c im Wesentlichen dadurch, dass das Endoskop 12b den Lichtwellenleiter 40b zum Leiten des Detektionslichts hat. Die Endoskophülle 24b gemäß dem dritten Ausführungsbeispiel nach Figur 3a und die Endoskophülle 24a dem zweiten Ausführungsbeispiel nach Figur 2a sind gleich ausgebildet.

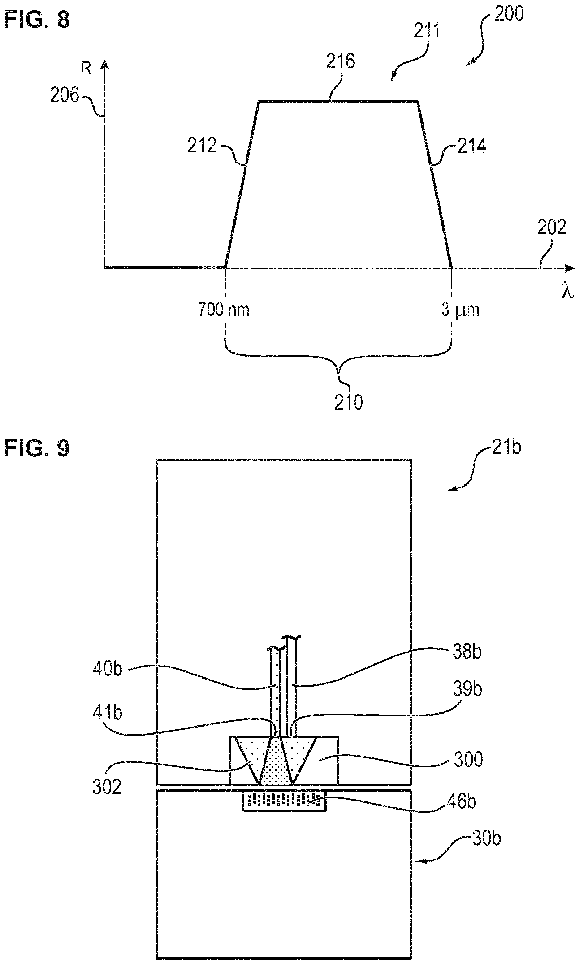

Das von der Detektionslichtquelle 42 emittierte Detektionslicht wird in den Lichtwellenleiter 40b eingekoppelt. Der Lichtwellenleiter 40b leitet das Detektionslicht zu der Spitze 21b am distalen Ende des Endoskopschafts 20b, wo es aus einem distalen Ende 41 des Lichtwellenleiters 40b austritt. Das austretende Detektionslicht wird durch das dem distalen Ende 41b des Lichtwellenleiters 40b gegenüberliegende lichtreflektierende Element 46b als Reflexionslicht in Richtung der Detektionsoptik 38b zurück reflektiert. Das Reflexionslicht wird durch die Detektionsoptik 38b von dem distalen Ende 39b der Detektionsoptik 38b bis zu dem Sensorelement 44 geleitet, der das Reflexionslicht erfasst. Die Reflektion des Detektionslichtes an der Spitze 21b wird nachfolgend anhand von Figur 9 noch näher erläutert.

Figur 3b zeigt eine schematische Darstellung einer Vorderansicht des stereoskopischen Endoskops 12b gemäß dem dritten Ausführungsbeispiel nach Figur 3a von einer distalen Seite her gesehen. Die Spitze 21b des Endoskops 12b gemäß dem dritten Ausführungsbeispiel nach Figur 3a unterscheidet sich von der Spitze 21a des Endoskops 12a gemäß dem zweiten Ausführungsbeispiel nach Figur 2a dadurch, dass neben dem distalen Ende 39b der Detektionsoptik 38b auch das distale Ende 41b des Lichtwellenleiters 40b an der Spitze 21b mittig angeordnet ist.

Figur 3c zeigt eine schematische Darstellung des optischen Elements 30b der sterilen Endoskophülle 14b gemäß dem dritten Ausführungsbeispiel nach den Figur 3a und 3b von einer proximalen Seite her gesehen. Das optische Element 30b der sterilen Endoskophülle 14b gemäß dem dritten Ausführungsbeispiel nach Figur 3a ist identisch zu dem optischen Element 30a der sterilen Endoskophülle 14a gemäß dem zweiten Ausführungsbeispiel nach Figur 2a ausgeführt.

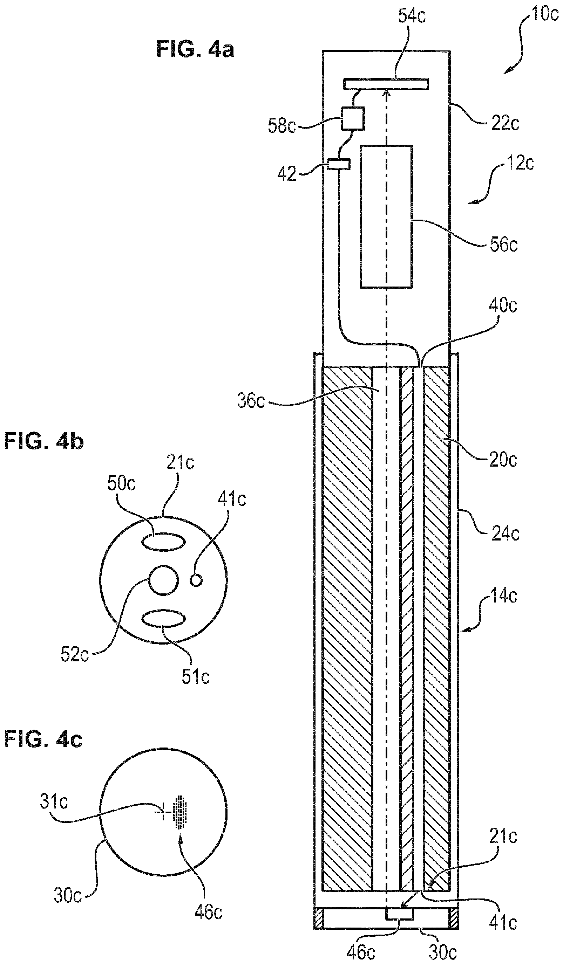

Figur 4a zeigt eine schematische Schnittdarstellung einer Anordnung 10c gemäß einem vierten Ausführungsbeispiel mit einem Monoendoskop 12c, das einen Lichtwellenleiter 40c zum Leiten des Detektionslichts hat, und mit der sterilen Endoskophülle 14c, die das an dem distalen Ende angeordnete optische Element 30c hat. Das nicht sterile Monoendoskop 12c ist in die sterile Endoskophülle 14c aufgenommen und durch diese gegenüber der Umgebung steril abgeschirmt.

Von der Endoskophülle 14c ist in Figur 4a nur ein vorderer Teil 24c gezeigt. Der übrige Aufbau der Endoskophülle 14c entspricht der Endoskophülle 14 nach Figur 1. Das distale Ende des vorderen Teils 24c der Endoskophülle 14c ist durch ein optisches Element 30c verschlossen. Auf der proximalen Seite des optischen Elements 30c hat dieses ein lichtreflektierendes Element 46c. Das lichtreflektierende Element 46c ist derart angeordnet, dass es einem Fenster 52c der Beobachtungsoptik 36c gegenüberliegt.

Das Endoskop 12c hat an einen in distale Richtung weisenden Endoskopschaft 20c, der den Lichtwellenleiter 40c und die Beobachtungsoptik 36c, die einen optischen Kanal umfasst. Das Endoskop 12c hat ferner einen an dem proximalen Ende des Endoskopschafts 20c angeordneten Endoskopkörper 22c, in dem insbesondere die Beleuchtungslichtquelle 42, ein Bildsensor 50c, eine Steuereinheit 56c und weitere optische Elemente, wie beispielsweise Prismen, Linsen oder Blenden, die der Beobachtungsoptik 36c angeordnet sind und die allgemein mit dem Bezugszeichen 56c bezeichnet sind, angeordnet sind.

Das von der Detektionslichtquelle 42 emittierte Detektionslicht wird in den Lichtwellenleiter 40c eingekoppelt. Der Lichtwellenleiter 40c leitet das Detektionslicht zu der an dem distalen Ende des Endoskopschafts 20c angeordneten Spitze 21c, wo es aus einem distalen Ende 41c des Lichtwellenleiters 40c austritt. Das austretende Detektionslicht wird durch das dem Fenster 52c der Beobachtungsoptik 36c gegenüberliegende lichtreflektierende Element 46c als Reflexionslicht in Richtung des Fensters 52c der Beobachtungsoptik 36c zurück reflektiert. Das Reflexionslicht wird durch die Beobachtungsoptik 36c und die der Beobachtungsoptik 36c zugeordneten optischen Elemente 56c bis zu dem Bildsensor 54c geleitet, der das Reflexionslicht erfasst.

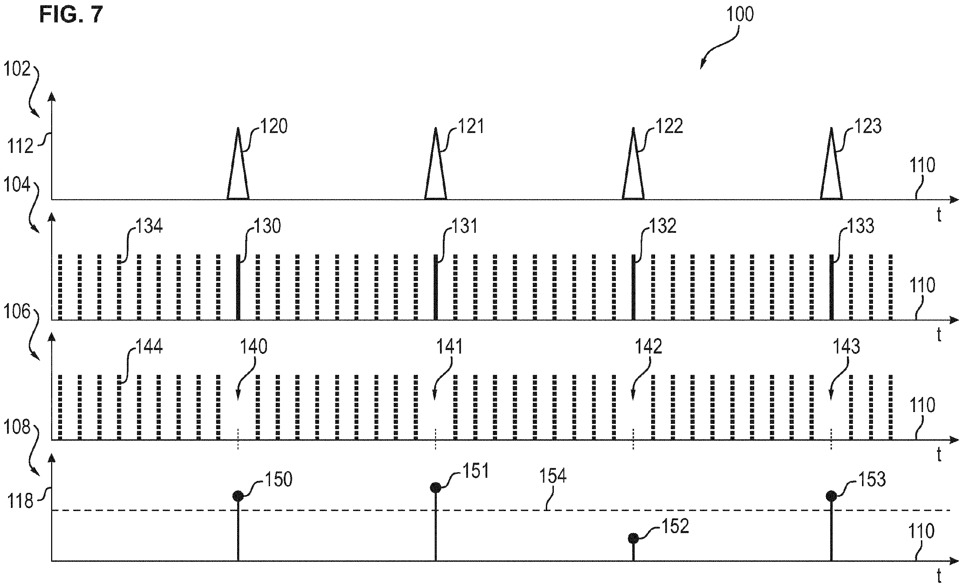

Die Steuereinheit 58c verarbeitet nur in einem ersten Betriebsmodus durch den Bildsensor 54c aufgenommene Bilder zur Bildanzeige weiter. Ferner steuert die Steuereinheit 58c die Detektionslichtquelle 42 derart, dass die Detektionslichtquelle 42 nur in einem zweiten Betriebsmodus das Detektionslicht emittiert. Dies ist weiter unten in Verbindung mit Figur 7 näher erläutert.

Figur 4b zeigt eine schematische Darstellung des Monoendoskops 12c gemäß dem vierten Ausführungsbeispiel nach Figur 4a von einer distalen Seite her gesehen. Das Ende des Lichtwellenleiters 41c ist in der Darstellung von Figur 4b rechts des mittig angeordneten Fensters 52c der Beobachtungsoptik 36c angeordnet. In der Darstellung von Figur 4b oberhalb und unterhalb des Fensters 52c ist je ein distales Ende 50c, 51c einer Beleuchtungsoptik zum Beleuchten des Bereichs distal der Spitze 21c angeordnet

Figur 4c zeigt eine schematische Darstellung des optischen Elements 30c der sterilen Endoskophülle 14c für das Monoendoskop 12c gemäß dem vierten Ausführungsbeispiel nach Figur 4a von einer proximalen Seite her gesehen. Das lichtreflektierende Element 46c ist rechts eines mittigen Punkts 31c, der in Figur 4c mit einem x markiert ist, auf der proximalen Seite, d.h. der Innenseite, des optischen Elements 30a angeordnet. Das lichtreflektierende Element 46c ist im vorliegenden Ausführungsbeispiel als lichtreflektierende Beschichtungen ausgebildet, die auf das optische Element 30c aufgebracht worden ist.

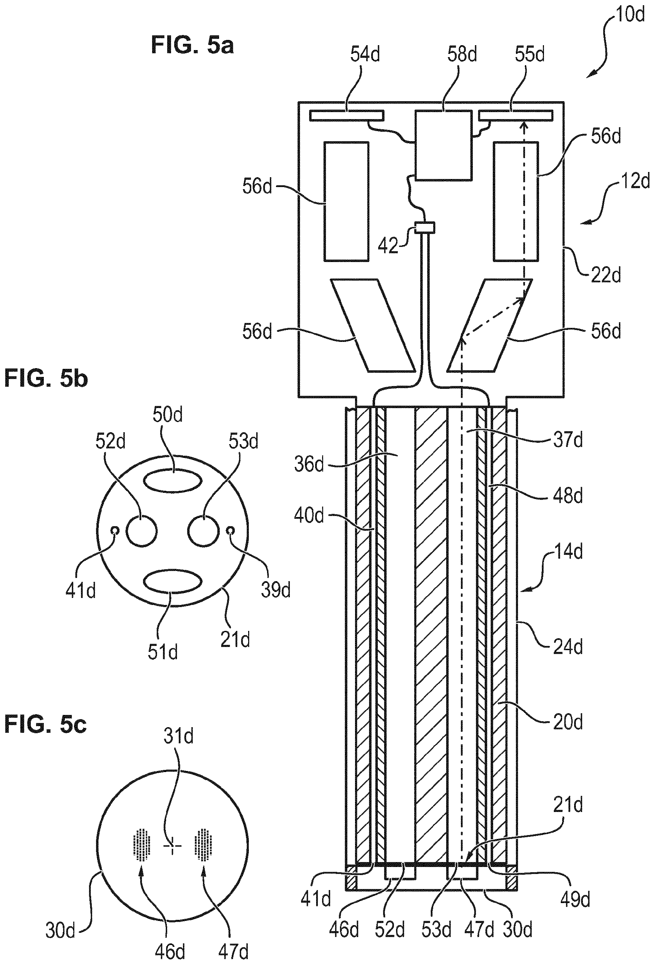

Figur 5a zeigt eine schematische Darstellung einer Anordnung 10d gemäß einem fünften Ausführungsbeispiel mit einem stereoskopischen Endoskop 12d, das zwei Lichtwellenleiter 40d, 48d zum Leiten des Detektionslichts hat Ferner umfasst die Anordnung 10d eine sterile Endoskophülle 14d, die ein an dem distalen Ende angeordnetes optisches Element hat. Das stereoskopische Endoskop 12d ist in der sterilen Endoskophülle 14d derart aufgenommen, dass es gegenüber der Umgebung steril abgeschirmt ist. Die Anordnung 10d gemäß dem fünften Ausführungsbeispiel nach Figur 5a unterscheidet sich von der Anordnung 10c gemäß dem vierten Ausführungsbeispiel nach Figur 4a im Wesentlichen durch den zweiten Lichtwellenleiter 48d.

Das Endoskop 12d hat an einen in distale Richtung weisenden Endoskopschaft 20d, der die Lichtwellenleiter 40d, 48d und eine Beobachtungsoptik 36d, 37d, die zwei optische Kanäle umfasst. Das Endoskop 12d hat ferner ein an dem proximalen Ende des Endoskopschafts 20d angeordneten Endoskopkörper 22d, in dem die Beleuchtungslichtquelle 42, ein erster Bildsensor 54d, ein zweiter Bildsensor 55d, eine Steuereinheit 58d und weitere optische Elemente, wie beispielsweise Prismen, Linsen oder Blenden, die der Beobachtungsoptik 36d, 37d zugeordnet sind und die allgemein mit dem Bezugszeichen 56d bezeichnet sind.

Von der Endoskophülle 14d ist in Figur 5a nur ein vorderer Teil 24d gezeigt. Der übrige Aufbau der Endoskophülle 14d entspricht der Endoskophülle 14 nach Figur 1. Ein distales Ende des vorderen Teils 24d der Endoskophülle 14d ist durch ein optisches Element 30d verschlossen. Auf der proximalen Seite des optischen Elements 30d sind ein erstes lichtreflektierendes Element 46d und ein zweites lichtreflektierendes Element 47d angeordnet. Das erste lichtreflektierende Element 46d ist derart angeordnet, dass es einem ersten Fenster 52d der Beobachtungsoptik 36d, 37d gegenüberliegt. Das zweite lichtreflektierende Element 47d ist derart angeordnet, dass es einem zweiten Fenster 53d der Beobachtungsoptik 36d, 37d gegenüberliegt.

Das von der Detektionslichtquelle 42 emittierte Detektionslicht wird in den ersten Lichtwellenleiter 40d eingekoppelt. Der erste Lichtwellenleiter 40d leitet das Detektionslicht zu der an dem distalen Ende des Endoskopschafts 20d angeordneten Spitze 21d, wo es aus einem distalen Ende 41d des ersten Lichtwellenleiters 40d austritt. Das austretende Detektionslicht wird durch das dem ersten Fenster 52d der Beobachtungsoptik 36d gegenüberliegende erste lichtreflektierende Element 46d als Reflexionslicht in Richtung des ersten Fensters 52d der Beobachtungsoptik 36d zurück reflektiert. Das Reflexionslicht wird durch die Beobachtungsoptik 36d und die der Beobachtungsoptik 36d zugeordneten optischen Elemente 56d bis zu dem ersten Bildsensor 54d geleitet, der das Reflexionslicht erfasst.

Das von der Detektionslichtquelle 42 ausgehendende Detektionslicht wird ferner in den zweiten Lichtwellenleiter 48d eingekoppelt. Der zweite Lichtwellenleiter 48d leitet das Detektionslicht zu der an dem distalen Ende des Endoskopschafts 20d angeordneten Spitze 21d, wo es aus einem distalen Ende 49d des zweiten Lichtwellenleiters 48d austritt. Das austretende Detektionslicht wird durch das dem zweiten Fenster 53d der Beobachtungsoptik 37d gegenüberliegende zweite lichtreflektierende Element 47d als Reflexionslicht in Richtung des zweiten Fensters 53d der Beobachtungsoptik 37d zurück reflektiert. Das Reflexionslicht wird durch die Beobachtungsoptik 37d und die der Beobachtungsoptik 37d zugeordneten optischen Elemente 56d bis zu dem zweiten Bildsensor 55d geleitet, der das Reflexionslicht erfasst.

Die Steuereinheit 58d verarbeitet nur in einem ersten Betriebsmodus durch den ersten bzw. den zweiten Bildsensor 54d, 55d aufgenommene Bilder zur Bildanzeige weiter. Die weiterverarbeiteten Bilder können über eine Ausgabeeinheit, beispielsweise einen Bildschirm, ausgegeben werden. Ferner steuert die Steuereinheit 58d die Detektionslichtquelle 42 derart, dass die Detektionslichtquelle 42 nur in einem zweiten Betriebsmodus das Detektionslicht emittiert. In dem zweiten Betriebsmodus werden die durch den ersten bzw. den zweiten Bildsensor 54d, 55d aufgenommene Bilder nicht zur Bildanzeige weiterverarbeitet. Die Steuereinheit 58d ermittelt in dem zweiten Betriebsmodus eine Intensität des Reflexionslichts aus den durch den ersten bzw. den zweiten Bildsensor 54d, 55d aufgenommenen Bildern. Liegt diese Intensität unterhalb eines voreingestellten Schwellenwertes, ist das Hinweis auf einen nicht korrekten Sitz der Endoskophülle 14d auf dem Endoskop 12d. Die Ausgabeeinheit kann in dem zweiten Betriebsmodus das letzte durch den ersten bzw. den zweiten Bildsensor 54d, 55d in dem ersten Betriebsmodus aufgenommene Bild ausgeben.

Figur 5b zeigt eine schematische Darstellung einer Vorderansicht des stereoskopischen Endoskops 12d gemäß dem fünften Ausführungsbeispiel nach Figur 5a von einer distalen Seite her gesehen. Die distalen Enden der zwei Lichtwellenleiter 41d, 39d sind in der Darstellung von Figur 5b links bzw. rechts des ersten bzw. des zweiten Fensters 52d, 53d der Beobachtungsoptik 36d, 37d angeordnet. In der Darstellung von Figur 5b sind oberhalb und unterhalb des ersten und des zweiten Fensters 52d, 53d je ein distales Ende 50d, 51d einer Beleuchtungsoptik zum Beleuchten des Bereichs distal der Spitze 21d angeordnet.

Figur 5c zeigt eine schematische Darstellung des optischen Elements 30d der sterilen Endoskophülle 14d gemäß dem fünften Ausführungsbeispiel nach Figur 5a von einer proximalen Seite her gesehen. Das erste lichtreflektierende Element 46d ist links eines mittig angeordneten Punkts 31d, der in Figur 5c mit einem x markiert ist, auf dem optischen Element 30d angeordnet. Das zweite lichtreflektierende Element 47d ist rechts des mittig angeordneten Punkts 31d auf dem optischen Element 30d angeordnet. Das erste und das zweite lichtreflektierende Element 46d, 47d sind in dem vorliegenden Ausführungsbeispiel als lichtreflektierende Beschichtungen ausgebildet, die auf das optische Element 30d aufgebracht worden sind.

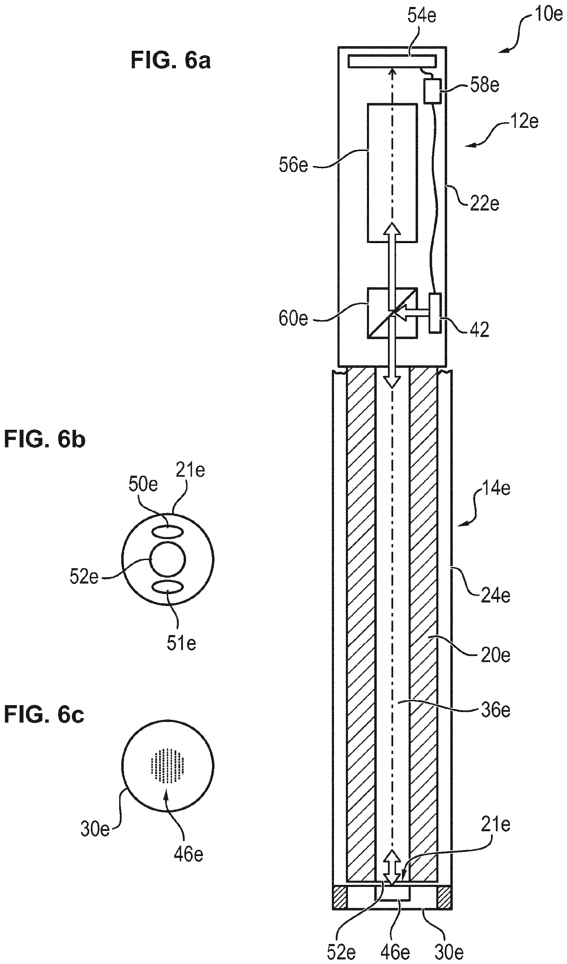

Figur 6a zeigt eine schematische Schnittdarstellung einer Anordnung 10e gemäß einem sechsten Ausführungsbeispiel. Die Anordnung 10e umfasst ein Monoendoskop 12e bestehend aus einem Endoskopkörper 22e und einem Endoskopschaft 20e, das einen Strahlteiler 60e zum Einkoppeln des Detektionslichts hat. Ferner hat die Anordnung 10e eine sterile Endoskophülle 14e, die ein an dem distalen Ende angeordnetes optisches Element hat. Das nicht sterile Monoendoskop 12e ist in der Endoskophülle 14e aufgenommen und dadurch gegenüber der Umgebung steril abgeschirmt. Die Anordnung 10e gemäß dem sechsten Ausführungsbeispiel nach Figur 6a unterscheidet sich von der Anordnung 10c gemäß dem fünften Ausführungsbeispiel nach Figur 5 im Wesentlichen dadurch, dass das Detektionslicht durch den Strahlteiler 60e in eine Beobachtungsoptik 36e des Monoendoskops 12e eingekoppelt wird.

Das von der Detektionslichtquelle 42 ausgehendende Detektionslicht wird mithilfe des Strahlteilers 60e in die Beobachtungsoptik 36e eingekoppelt. Die Beobachtungsoptik 36e leitet das Detektionslicht zu der an dem distalen Ende des Endoskopschafts 20e angeordneten Spitze 21e, wo es aus einem Fenster 52e einer Beobachtungsoptik 36e austritt. Das austretende Detektionslicht wird durch das dem Fenster 52e der Beobachtungsoptik 36e gegenüberliegende lichtreflektierende Element 46e als Reflexionslicht in Richtung des Fensters 52e der Beobachtungsoptik 36e zurück reflektiert. Das Reflexionslicht wird durch die Beobachtungsoptik 36e und die der Beobachtungsoptik 36e zugeordneten optischen Elemente 56e bis zu dem Bildsensor 54e geleitet, der das Reflexionslicht erfasst.

Die Steuereinheit 58e verarbeitet nur in einem ersten Betriebsmodus durch den Bildsensor 54e aufgenommene Bilder zur Bildanzeige weiter. Ferner steuert die Steuereinheit 58e die Detektionslichtquelle 42 derart, dass die Detektionslichtquelle 42 nur in einem zweiten Betriebsmodus das Detektionslicht emittiert. In dem zweiten Betriebsmodus werden die durch den Bildsensor 54e aufgenommene Bilder nicht zur Bildanzeige weiterverarbeitet. Die Steuereinheit 58e ermittelt in dem zweiten Betriebsmodus eine Intensität des Reflexionslichts aus den durch den Bildsensor 54e aufgenommenen Bildern. Liegt diese Intensität unterhalb eines voreingestellten Schwellenwertes, ist das Hinweis auf einen nicht korrekten Sitz der Endoskophülle 14e auf dem Endoskop 12e. Die Ausgabeeinheit kann in dem zweiten Betriebsmodus das letzte durch den Bildsensor 54e in dem ersten Betriebsmodus aufgenommene Bild ausgeben.

Figur 6b zeigt eine schematische Darstellung des nicht sterilen Monoendoskops 12e gemäß dem sechsten Ausführungsbeispiel nach Figur 6a von einer distalen Seite her gesehen. Die Spitze 21e des Endoskops 12e nach Figur 6a unterscheidet sich von der Spitze 21c des Endoskops 12c nach Figur 4a dadurch, dass die Spitze 21e des Endoskops 12e nach Figur 6a kein distales Ende eines Lichtwellenleiters aufweist, da das Reflexionslicht durch die Beobachtungsoptik 36e und die der Beobachtungsoptik 36e zugeordneten optischen Elemente 56e geleitet wird. In der Darstellung von Figur 6b ist oberhalb und unterhalb des Fensters 52e je ein distales Ende 50d, 51d einer Beleuchtungsoptik zum Beleuchten des Bereichs distal der Spitze 21d angeordnet.

Figur 6c zeigt eine schematische Darstellung des optischen Elements 30e der sterilen Endoskophülle 14e gemäß dem sechsten Ausführungsbeispiel nach Figur 6a von einer proximalen Seite her gesehen. Das optische Element 30e der sterilen Endoskophülle 14e gemäß dem sechsten Ausführungsbeispiel nach Figur 6a ist identisch zu dem optischen Element 30a der sterilen Endoskophülle 14a gemäß dem zweiten Ausführungsbeispiel nach Figur 2a ausgeführt.

Figur 7 zeigt ein Diagramm 100 zur Erläuterung der Funktionsweise der Anordnung 10c zur sterilen Handhabung eines nicht sterilen Endoskops gemäß dem vierten Ausführungsbeispiel nach den Figuren 4a bis 4c. Das Diagramm 100 umfasst vier Unterdiagramme 102, 104, 106, 108. Die vier Unterdiagramme 102, 104, 106, 108 haben jeweils eine Zeitachse 110, die von links nach rechts einen zeitlichen Verlauf angibt.

Das erste Unterdiagramm 102 hat eine Ordinatenachse 112, welche die Intensität des von der Detektionslichtquelle 42 in dem ersten Betriebsmodus emittierten Beleuchtungslichts angibt. Beispielhaft ist eine regelmäßige Folge von vier Detektionslichtpulsen 120, 121, 122, 123 gezeigt.

Das zweite Unterdiagramm 104 gibt an, zu welchen Zeitpunkten der Bildsensor 54c entweder das Detektionslicht und das Umgebungslicht oder nur das Umgebungslicht erfasst. Zeitpunkte, zu denen der Bildsensor 54c das Detektionslicht und das Umgebungslicht erfasst, sind als Impulse 130, 131, 132, 133 in dem zweiten Unterdiagramm 104 dargestellt. Diese Zeitpunkte entsprechen den Zeitpunkten, an denen die Detektionslichtquelle 42 die Detektionslichtpulse 120, 121, 122, 123 emittiert. Zeitpunkte, zu denen der Bildsensor 54c nur das Umgebungslicht erfasst, sind als gepunktete Impulse in dem zweiten Unterdiagramm 104 dargestellt, von denen beispielhaft ein Impuls mit dem Bezugszeichen 134 versehen ist. Diese Zeitpunkte entsprechen den Zeitpunkten, an denen die Detektionslichtquelle 42 kein Detektionslicht emittiert

Das dritte Unterdiagramm 106 gibt an, zu welchen Zeitpunkten Steuereinheit 58c die durch den Bildsensor 54c aufgenommenen Bilder in dem zweiten Betriebsmodus zur Bildanzeige weiterverarbeitet. Diese Zeitpunkte sind als Impulse in dem dritten Unterdiagramm 106 dargestellt, von denen beispielhaft ein Impuls mit dem Bezugszeichen 144 versehen ist. Zu den Zeitpunkten an denen der Bildsensor 54c das Detektionslicht und das Umgebungslicht erfasst, gibt die Steuereinheit 58c das von dem Bildsensor 54c aufgenommene Bild nicht zur Bildanzeige weiter, sondern ermittelt eine Intensität des Reflexionslichts aus den durch den Bildsensor 54c aufgenommenen Bildern. Liegt diese Intensität unterhalb eines voreingestellten Schwellenwertes, ist das Hinweis auf einen nicht korrekten Sitz der Endoskophülle 14c auf dem Endoskop 12c. Die Ausgabeeinheit kann im zweiten Betriebsmodus das letzte durch den Bildsensor 54c in dem ersten Betriebsmodus aufgenommene Bild ausgeben. Diese Zeitpunkte sind in dem dritten Unterdiagramm 106 mit den Bezugszeichen 140, 141, 142, 143 bezeichnet.

Das vierte Unterdiagramm 108 hat eine Ordinatenachse 118, welche die Intensität des von dem Bildsensor 54c erfassten Beleuchtungslichts angibt, die durch die Steuereinheit 58c ermittelt wird. In dem vierten Unterdiagramm 108 sind vier Intensitätswerte 150, 151, 152, 153 gezeigt, die den Detektionslichtpulsen 120, 121, 122, 123 zugeordnet sind. Das vierte Unterdiagramm 108 zeigt ferner eine waagerechte gestrichelte Linie 154, die den voreingestellten Schwellenwert der ermittelten Intensität des durch den Bildsensor 54c erfassten Detektionslichts anzeigt. Die von links gesehen ersten zwei Intensitätswerte 150, 151 und der letzte Intensitätswert 153 liegen oberhalb des Schwellenwertes. Der von links gesehen dritte Intensitätswert 152 liegt unterhalb des Schwellenwertes, was auf einen nicht korrekten Sitz der Endoskophülle 14c hinweist.

Das in Verbindung mit der Anordnung 10c nach Figur 4 beschriebene Verfahren kann alternativ auch mit den Anordnungen 10d oder 10e nach den Figuren 5 oder 6 durchgeführt werden.

Figur 8 zeigt ein Diagramm 200 eines Wellenlängenbereichs 210 des Detektionslichts der Anordnung 10 zur sterilen Handhabung eines nicht sterilen Endoskops 12 gemäß dem ersten Ausführungsbeispiel nach Figur 1. Der Wellenlängenbereich 210 ist nur beispielhaft für die Anordnung 10 nach Figur 1 illustriert. Bei anderen Ausführungsbeispielen kann der Wellenlängenbereich abweichen.

Das Diagramm 200 hat eine Abszissenachse 202, auf der die Wellenlänge λ angegeben ist, und eine Ordinatenachse 204, welche eine Intensität R angibt. Der Wellenlängenbereich 210 ist als eine Intensitätskurve 211 gezeigt. Die Intensitätskurve 211 weist eine erste Flanke 212 bei einer Wellenlänge λ von 700 nm auf, d.h. die Intensität R des Detektionslichts nimmt ab einer Wellenlänge λ von 700 nm zu. Die Intensitätskurve 211 weist ferner eine zweite Flanke 214 bei einer Wellenlänge λ von 3 µm auf, d.h. die Intensität R des Detektionslichts nimmt bis zu einer Wellenlänge λ von 3 µm ab. Zwischen der ersten und der zweiten Flanke 212, 214 hat die Intensitätsverteilung 211 ein Plateau 216. Der Wellenlängenbereich 210 des Detektionslichts umfasst folglich Wellenlängen λ von 700 nm bis 3 µm, was nahinfraroten Licht entspricht. Alternativ kann das Detektionslicht auch ultraviolettes Licht, d.h. das Detektionslicht umfasst Wellenlängen λ zwischen 280 nm und 400 nm, oder infrarotes Licht, d.h. das Detektionslicht umfasst Wellenlängen λ zwischen 800 nm und 1 mm, sein.

Figur 9 zeigt ein distales Ende 21b des Endoskops 12b gemäß dem dritten Ausführungsbeispiel nach den Figuren 3a bis 3c in einer schematischen Schnittdarstellung. Das distale Ende 21b des Endoskops 12b weist das distale Ende 39 der Detektionsoptik 38 und ein distales Ende 41b eines Lichtwellenleiters 40b auf. Das distale Endes 39b der Detektionsoptik 38b und das distale Ende 41b des Lichtwellenleiters 40b sind innerhalb einer Ausnehmung 300 angeordnet. Aus dem distalen Ende 41b des Lichtwellenleiters 40b austretendes Detektionslicht 302 wird durch das der Ausnehmung 300 gegenüberliegend auf dem optischen Element 30b angeordneten lichtreflektierenden Element 46 in Richtung des distalen Endes 39b der Detektionsoptik 38b zurückreflektiert. Durch die Ausnehmung 300 wird der Abstand zwischen dem distalen Ende 41b des Lichtwellenleiters 40b und dem lichtreflektierenden Element 46b vergrößert, so dass sich das Lichtbündel des austretenden Detektionslichts 302 verbreitert. Alternativ oder zusätzlich kann die numerische Apertur des Lichtwellenleiters 40b angepasst werden, um eine Verbreiterung des Lichtbündels zu erreichen.

### Bezugszeichenliste

- 10: Anordnung
- 12: Endoskop
- 14: Endoskophülle
- 20: Endoskopschaft
- 22: Endoskopkörper
- 24: vorderer Teil
- 26: Mittelteil
- 28: Verschlusselement
- 29: Führungssteg
- 30, 56: optisches Element
- 31: mittiger Punkt
- 32: Zuführ- und Entnahmeöffnung
- 34: Kontaktbereich
- 36, 37: Beobachtungsoptik
- 38: Detektionsoptik
- 21, 39, 41, 50, 51: distales Ende
- 40,48: Lichtwellenleiter
- 42: Detektionslichtquelle
- 44: Sensorelement
- 46: lichtreflektierendes Element
- 52, 53: Fenster
- 54, 55: Bildsensor
- 58: Steuereinheit
- 60: Strahlteiler
- 100,200: Diagramm
- 102, 104, 106, 108: Unterdiagramm
- 110: Zeitachse
- 112, 118, 204: Ordinatenachse
- 120-123: Detektionslichtpulse
- 130-133, 140-143, 154: Impulse
- 150-153: Intensitätswerte
- 202: Abszissenachse
- 210: Wellenlängenbereich
- 211: Intensitätskurve
- 212,214: Flanke
- 216: Plateau
- 300: Ausnehmung
- 302: austretendes Detektionslicht

## Patentansprüche

1. Anordnung zur sterilen Handhabung eines nicht sterilen Endoskops in einer sterilen Umgebung,
mit einer sterilen Endoskophülle (14), die ein an einem distalen Ende der Endoskophülle (14) angeordnetes optisches Element (30) hat,
wobei das optische Element (30) ein lichtreflektierendes Element (46) oder einen lichtreflektierenden Bereich aufweist, das bzw. der von einer Detektionslichtquelle (42) emittiertes Detektionslicht als Reflexionslicht in Richtung eines proximalen Endes der Endoskophülle (14) reflektiert; und
mit einem nicht sterilen Endoskop (12), das einen Endoskopschaft (20) umfasst,
wobei das Endoskop in der Endoskophülle (14) aufgenommen ist und durch diese gegenüber der Umgebung steril abgeschirmt ist,
wobei
das Endoskop ferner eine Beleuchtungsoptik eingerichtet zum Leiten von Beleuchtungs-
licht und eine Beobachtungsoptik (36, 37) eingerichtet zur Detektion und/oder Weiter-
leitung von Umgebungslicht, das in ein distales Ende des Endoskopschafts (20) eintritt, umfasst, **dadurch gekennzeichnet,**
**dass** das Endoskop (12) eine von der Beleuchtungsoptik und der Beobachtungsoptik (36, 37) verschiedene Detektionsoptik (38) eingerichtet zum Leiten des von der Detektionslichtquelle (42) emittierten Detektionslichts hat.

2. Anordnung nach Anspruch 1, **dadurch gekennzeichnet, dass** das lichtreflektierende Element (46) oder der lichtreflektierende Bereich in wenigstens einem optischen Wellenlängenbereich außerhalb des Wellenlängenbereichs (210) des Detektionslichts transparent ist.

3. Anordnung nach Anspruch 1 oder 2, **dadurch gekennzeichnet, dass** das lichtreflektierende Element (46) oder der lichtreflektierende Bereich Licht in einem ultravioletten (UV), infraroten (IR) oder nahinfraroten (NIR) Wellenlängenbereich reflektiert.

4. Anordnung nach einem der vorhergehenden Ansprüche, **dadurch gekennzeichnet, dass** das lichtreflektierende Element (46) oder der lichtreflektierende Bereich auf einer dem proximalen Ende der Endoskophülle (14) zugewandten Seite des optischen Elements (30) angeordnet ist.

5. Anordnung nach einem der vorhergehenden Ansprüche, **dadurch gekennzeichnet, dass** die Beobachtungsoptik (36, 37) das Reflexionslicht leitet.

6. Anordnung nach einem der vorhergehenden Ansprüche, **dadurch gekennzeichnet, dass** das Endoskop (12) einen Strahlteiler (60) hat, der das Detektionslicht in die Beleuchtungsoptik oder die Detektionsoptik (38) einkoppelt.

7. Anordnung nach Anspruch 6, **dadurch gekennzeichnet, dass** der Strahlteiler (60) das Reflexionslicht aus der Detektionsoptik (38) oder der Beobachtungsoptik (36, 37) auskoppelt, so dass es auf ein Sensorelement (44), insbesondere einen Photodetektor oder einen CCD-Sensor, trifft.

8. Anordnung nach einem der vorhergehenden Ansprüche- , **dadurch gekennzeichnet, dass** das Endoskop das oder ein Sensorelement, insbesondere einen Photodetektor oder einen CCD-Chip, umfasst, welches das Reflexionslicht erfasst.

9. Anordnung nach einem der Ansprüche 1 bis 6, **dadurch gekennzeichnet, dass** die Beobachtungsoptik einen Bildsensor (54) umfasst, der das in das distale Ende des Endoskopschafts (20) eintretende Umgebungslicht erfasst und der das Reflexionslicht erfasst.

10. Anordnung nach Anspruch 9, **dadurch gekennzeichnet, dass** die Anordnung (10) eine Steuereinheit (58) umfasst, dass die Steuereinheit (58) nur in einem ersten Betriebsmodus durch den Bildsensor (54) aufgenommene Bilder zur Bildanzeige weiterverarbeitet, und dass die Steuereinheit (58) die Detektionslichtquelle (42) derart steuert, dass die Detektionslichtquelle (42) nur in einem zweiten Betriebsmodus das Detektionslicht emittiert, wobei der Bildsensor im zweiten Betriebsmodus das von dem lichtreflektierenden Element reflektierte Reflexionslicht detektiert.

11. Anordnung nach einem der vorhergehenden Ansprüche, **dadurch gekennzeichnet, dass** die Detektionslichtquelle (42) das Beleuchtungslicht emittiert und dass das Detektionslicht aus dem Beleuchtungslicht ausgekoppelt wird.

12. Anordnung nach einem der Ansprüche 1 bis 10, **dadurch gekennzeichnet, dass** die Detektionslichtquelle (42) Detektionslicht in einem ultravioletten, infraroten oder nahinfraroten Wellenlängenbereich (210) emittiert.

13. Anordnung nach einem der Ansprüche 1 bis 10, **dadurch gekennzeichnet, dass** die Detektionslichtquelle (42) kohärentes Detektionslicht emittiert.

## Claims

1. An arrangement for the sterile handling of a non-sterile endoscope in a sterile environment,
with a sterile endoscope sheath (14) having an optical element (30) arranged at a distal end of the endoscope sheath (14),
wherein the optical element (30) has a light-reflecting element (46) or a light-reflecting area which reflects detection light emitted from a detection light source (42) as reflection light toward a proximal end of the endoscope sheath (14); and
with a non-sterile endoscope (12) comprising an endoscope shaft (20), wherein the endoscope is received in the endoscope sheath (14) and is shielded by it in a sterile manner against the environment,
wherein the endoscope further comprises an illumination optical system configured to guide illumination light and an observation optical system (36, 37) configured to detect and/or forward ambient light entering a distal end of the endoscope shaft (20),
**characterized in that** the endoscope (12) has a detection optical system (38) different from the illumination optical system and the observation optical system (36, 37) and configured to guide the detection light emitted by the detection light source (42).

2. The arrangement according to claim 1, **characterized in that** the light-reflecting element (46) or the light-reflecting area is transparent in at least one optical wavelength range outside the wavelength range (210) of the detection light.

3. The arrangement according to claim 1 or 2, **characterized in that** the light-reflecting element (46) or the light-reflecting area reflects light in an ultraviolet (UV), an infrared (IR) or a near-infrared (NIR) wavelength range.

4. The arrangement according to any one of the preceding claims, **characterized in that** the light-reflecting element (46) or the light-reflecting area is arranged on a side of the optical element (30) facing the proximal end of the endoscope sheath (14).

5. The arrangement according to any one of the preceding claims, **characterized in that** the observation optical system (36, 37) guides the reflection light.

6. The arrangement according to any one of the preceding claims, **characterized in that** the endoscope (12) has a beam splitter (60) which couples the detection light into the illumination optical system or the detection optical system (38).

7. The arrangement according to claim 6, **characterized in that** the beam splitter (60) couples the reflection light out of the detection optical system (38) or the observation optical system (36, 37) so that it is incident on a sensor element (44), in particular a photodetector or a CCD sensor.

8. The arrangement according to any one of the preceding claims, **characterized in that** the endoscope comprises the or a sensor element, in particular a photodetector or a CCD chip, which detects the reflection light.

9. The arrangement according to one of the claims 1 to 6, **characterized in that** the observation optical system comprises an image sensor (54) which detects the ambient light entering the distal end of the endoscope shaft (20) and the reflection light.

10. The arrangement according to claim 9, **characterized in that** the arrangement (10) comprises a control unit (58), that the control unit (58) further processes images captured by the image sensor (54) for image display only in a first operating mode, and that the control unit (58) controls the detection light source (42) such that the detection light source (42) emits the detection light only in a second operating mode, wherein the image sensor detects the reflection light reflected by the light-reflecting element in the second operating mode.

11. The arrangement according to any one of the preceding claims, **characterized in that** the detection light source (42) emits the illumination light and that the detection light is coupled out of the illumination light.

12. The arrangement according to one of the claims 1 to 10, **characterized in that** the detection light source (42) emits detection light in an ultraviolet, infrared, or near-infrared wavelength range (210).

13. - The arrangement according to one of the claims 1 to 10, **characterized in that** the detection light source (42) emits coherent detection light.

## Revendications

1. Dispositif permettant une manipulation stérile d'un endoscope non stérile dans un environnement stérile,
avec une gaine d'endoscope stérile (14) présentant un élément optique (30) agencé à une extrémité distale de la gaine d'endoscope (14),
dans lequel l'élément optique (30) présente un élément réfléchissant la lumière (46), ou une région réfléchissant la lumière, qui réfléchit la lumière de détection, émise par une source de lumière de détection (42), sous forme de lumière de réflexion vers une extrémité proximale de la gaine d'endoscope (14) ; et
avec un endoscope non stérile (12) comprenant une tige d'endoscope (20),
dans lequel l'endoscope est accueilli dans la gaine d'endoscope (14) et est protégé de manière stérile par rapport à l'environnement grâce à ladite gaine d'endoscope,
dans lequel
l'endoscope comprend en outre une optique d'éclairage conçue pour guider la lumière d'éclairage et une optique d'observation (36, 37) conçue pour détecter et/ou transmettre la lumière ambiante qui rentre par une extrémité distale de la tige d'endoscope (20), **caractérisé en ce que**
l'endoscope (12) présente une optique de détection (38) différente de l'optique d'éclairage et de l'optique d'observation (36, 37) et conçue pour guider la lumière de détection émise par la source de lumière de détection (42).

2. Dispositif selon la revendication 1, **caractérisé en ce que** l'élément réfléchissant la lumière (46) ou la région réfléchissant la lumière est transparent(e) dans au moins une plage de longueurs d'onde optique située en dehors de la plage de longueurs d'onde (210) de la lumière de détection.

3. Dispositif selon la revendication 1 ou 2, **caractérisé en ce que** l'élément réfléchissant la lumière (46) ou la région réfléchissant la lumière réfléchit la lumière dans une plage de longueurs d'onde de l'ultraviolet (UV), de l'infrarouge (IR) ou du proche infrarouge (NIR).

4. Dispositif selon l'une quelconque des revendications précédentes, **caractérisé en ce que** l'élément réfléchissant la lumière (46) ou la région réfléchissant la lumière est agencé (e) sur un côté de l'élément optique (30) qui est tourné vers l'extrémité proximale de la gaine d'endoscope (14) .

5. Dispositif selon l'une quelconque des revendications précédentes, **caractérisé en ce que** l'optique d'observation (36, 37) guide la lumière de réflexion.

6. Dispositif selon l'une quelconque des revendications précédentes, **caractérisé en ce que** l'endoscope (12) présente un diviseur de faisceau (60) qui injecte la lumière de détection dans l'optique d'éclairage ou l'optique de détection (38).

7. Dispositif selon la revendication 6, **caractérisé en ce que** le diviseur de faisceau (60) extrait la lumière de réflexion de l'optique de détection (38) ou de l'optique d'observation (36, 37) de sorte qu'elle rencontre un élément formant capteur (44), en particulier un photodétecteur ou un capteur CCD.

8. Dispositif selon l'une quelconque des revendications précédentes, **caractérisé en ce que** l'endoscope comprend l'élément formant capteur ou un élément formant capteur, en particulier un photodétecteur ou une puce CCD, qui détecte la lumière de réflexion.

9. Dispositif selon l'une quelconque des revendications 1 à 6, **caractérisé en ce que** l'optique d'observation comprend un capteur d'image (54) qui détecte la lumière ambiante rentrant par l'extrémité distale de la tige d'endoscope (20) et qui détecte la lumière de réflexion.

10. Dispositif selon la revendication 9, **caractérisé en ce que** le dispositif (10) comprend une unité de commande (58), **en ce que** l'unité de commande (58) ne traite, en vue de l'affichage des images, que des images recueillies grâce au capteur d'image (54) dans un premier mode de fonctionnement, et **en ce que** l'unité de commande (58) commande la source de lumière de détection (42) de telle manière que la source de lumière de détection (42) n'émet la lumière de détection que dans un second mode de fonctionnement, dans lequel, dans le second mode de fonctionnement, le capteur d'image détecte la lumière de réflexion réfléchie par l'élément réfléchissant la lumière.

11. Dispositif selon l'une quelconque des revendications précédentes, **caractérisé en ce que** la source de lumière de détection (42) émet la lumière d'éclairage et **en ce que** la lumière de détection est extraite de la lumière d'éclairage.

12. Dispositif selon l'une quelconque des revendications 1 à 10, **caractérisé en ce que** la source de lumière de détection (42) émet de la lumière de détection dans une plage de longueurs d'onde (210) de l'ultraviolet, de l'infrarouge ou du proche infrarouge.

13. Dispositif selon l'une quelconque des revendications 1 à 10, **caractérisé en ce que** la source de lumière de détection (42) émet une lumière de détection cohérente.
